# EUROPEAN PATENT APPLICATION

(11) **EP 3 872 131 A1**
(43) Date of publication of application: **01.09.2021**
(21) Application number: 19874985.5
(22) Date of filing: 24.10.2019
(51) Int. Cl.: C08L 33/02, A61K 8/34, A61K 8/81, A61Q 19/00, C08K 5/053

(54) **GEL COMPOSITION, DISPERSION, AND METHOD FOR PRODUCING GEL COMPOSITION**

(30) Priority: 26.10.2018 JP 2018202228
(71) Applicant: Sumitomo Seika Chemicals Co., Ltd., Kako-gun, Hyogo 675-0145 (JP)
(72) Inventor: MIYAMOTO, Makiko, Himeji-shi, Hyogo 672-8076 (JP); YAMAGUCHI, Hirofumi, Himeji-shi, Hyogo 672-8076 (JP)
(74) Representative: Vossius & Partner Patentanwälte Rechtsanwälte mbB
(86) International application number: PCT/JP2019/041702
(87) International publication number: WO 2020/085431

(57) **Abstract**

Provided are a gelled composition containing a thickening agent homogeneously dissolved in a polyhydric alcohol and a method for producing the gelled composition. The gelled composition according to the present invention contains a polymer containing an ethylenically unsaturated carboxylic acid unit and an anhydrous polyhydric alcohol, wherein the polymer is a partially neutralized product that has a degree of neutralization of 20 to 75%, and the polymer is dissolved in the anhydrous polyhydric alcohol. The method for producing the gelled composition according to the present invention includes a first step of preparing a dispersion of a polymer containing an ethylenically unsaturated carboxylic acid unit in an anhydrous polyhydric alcohol, and a second step of mixing the dispersion with an anhydrous polyhydric alcohol to prepare a solution of the polymer in the polyhydric alcohol, wherein the polymer for use in the first step is a partially neutralized product that has a degree of neutralization of 20 to 75%, and the polymer for use in the first step is in the form of spherical particles.

## Description

### Technical Field

The present invention relates a gelled composition, a dispersion, and a method for producing a gelled composition.

### Background Art

Gelled materials have a wide range of applications, such as warm gels, scrub cosmetics, sheet masks, and nourishing cosmetic materials. These gelled materials typically contain a thickening agent, which imparts a thickening effect to a range of materials.

A variety of materials are known as a thickening agent. Examples include natural thickening agents, such as xanthan gum and guar gum; semisynthetic thickening agents, such as hydroxyethyl cellulose and carboxy methylcellulose; and synthetic thickening agents, such as carboxyvinyl polymers and polyethylene oxide. Of these thickening agents, carboxyvinyl polymers are heavily used due to their low price, strong thickening effect, and ability to form a gel in small amounts.

Gelled materials vary in type, such as those containing water as the main component and those containing a polyhydric alcohol (e.g., glycerol) as the main component. Thus, the type of thickening agent is appropriately designed according to their formulation. For example, PTL 1 suggests a gelled material that contains an alkyl-modified carboxy vinyl polymer as a thickening agent.

### Citation List

Patent Literature

PTL 1: JP2010-37272A

### Summary of Invention

### Technical Problem

However, while conventional thickening agents can be homogeneously dispersed in water, they easily form aggregates *(mamako)* in a polyhydric alcohol, taking a long time in forming a homogeneous thickening liquid. Thus, it has been considered difficult to provide a water-free polyhydric alcohol thickening liquid (i.e., a gelled material that contains an anhydrous polyhydric alcohol as a base) by using traditional techniques. However, from the standpoint of, for example, effectively producing a warming effect, various fields such as the field of cosmetic materials desire a gelled material that contains an anhydrous polyhydric alcohol as a base without containing water; there has been demand for the establishment of a technique to obtain a gelled material by using a thickening agent that easily brings about a thickening effect on anhydrous polyhydric alcohols.

The present invention was made in view of the current circumstances described above. An object of the present invention is to provide a gelled composition that contains a thickening agent homogeneously dissolved in a polyhydric alcohol and a method for producing the gelled composition. Another object of the invention is to provide a dispersion suitable as a starting material for producing the gelled composition.

### Solution to Problem

The present inventors conducted extensive research to achieve the objects and found that the objects can be achieved by using a thickening agent that contains a polymer containing an ethylenically unsaturated carboxylic acid unit with a specific degree of neutralization. They then completed the present invention.

Specifically, the present invention includes, for example, the subject matter described in the following items.

### Item 1

A gelled composition comprising
a polymer containing an ethylenically unsaturated carboxylic acid unit, and
an anhydrous polyhydric alcohol, wherein the polymer is a partially neutralized product that has a degree of neutralization of 20 to 75%, and the polymer is dissolved in the anhydrous polyhydric alcohol.

### Item 2

The gelled composition according to Item 1, wherein the polymer is present in an amount of 0.05 to 0.95% based on the total mass of the anhydrous polyhydric alcohol and the polymer.

### Item 3

A dispersion comprising
a polymer containing an ethylenically unsaturated carboxylic acid unit, and
an anhydrous polyhydric alcohol, wherein the polymer is a partially neutralized product that has a degree of neutralization of 20 to 75%, the polymer is in the form of spherical particles, and the polymer is dispersed in the anhydrous polyhydric alcohol.

### Item 4

The dispersion according to Item 3, wherein the polymer is present in an amount of 1.0 to 16.0 mass% based on the total mass of the anhydrous polyhydric alcohol and the polymer.

### Item 5

A method for producing the gelled composition of Item 1 or 2, the method comprising
a first step of preparing a dispersion of a polymer containing an ethylenically unsaturated carboxylic acid unit in an anhydrous polyhydric alcohol, and
a second step of mixing the dispersion with an anhydrous polyhydric alcohol to prepare a solution of the polymer in the polyhydric alcohol, wherein the polymer for use in the first step is a partially neutralized product that has a degree of neutralization of 20 to 75%, and the polymer for use in the first step is in the form of spherical particles.

### Item 6

The method according to Item 5, wherein in the first step, the proportion of the polymer for use is 1.0 to 16.0 mass% based on the total mass of the anhydrous polyhydric alcohol and the polymer.

### Advantageous Effects of Invention

The gelled composition according to the present invention contains a thickening agent homogeneously dissolved in a polyhydric alcohol.

Because of its ability to make it easier to produce a gelled composition, the dispersion according to the present invention is suitable as a starting material for producing the gelled composition.

The method for producing a gelled composition according to the present invention makes it easier to produce a gelled composition that contains a thickening agent homogeneously dissolved in a polyhydric alcohol.

### Description of Embodiments

The following describes embodiments of the present invention in detail. In the present specification, the terms "comprise," "contain," and "include" include the concepts of comprising, containing, including, consisting essentially of, and consisting of.

The gelled composition according to the present invention contains a polymer containing an ethylenically unsaturated carboxylic acid unit and an anhydrous polyhydric alcohol. The polymer in the gelled composition is a partially neutralized product that has a degree of neutralization of 20 to 75%, and the polymer is dissolved in the anhydrous polyhydric alcohol.

The dispersion according to the present invention contains a polymer containing an ethylenically unsaturated carboxylic acid unit and an anhydrous polyhydric alcohol. The polymer in the dispersion is a partially neutralized product that has a degree of neutralization of 20 to 75%. The polymer is in the form of spherical particles and is dispersed in the anhydrous polyhydric alcohol.

### Polymer

In the present invention, the polymer contains at least an ethylenically unsaturated carboxylic acid unit as a repeating structural unit. Just to note, the ethylenically unsaturated carboxylic acid unit refers to a repeating structural unit formed when an ethylenically unsaturated carboxylic acid monomer is polymerized, and does not refer to the monomer itself.

The ethylenically unsaturated carboxylic acid monomer can be of any type. Examples include α,β-unsaturated carboxylic acids such as (meth)acrylic acid, crotonic acid, maleic acid, fumaric acid, and itaconic acid. These ethylenically unsaturated carboxylic acid monomers may be used singly or in a combination of two or more. In this specification, "(meth)acrylic" means "acrylic or methacrylic." For example, "(meth)acrylic acid" is synonymous with "acrylic acid or methacrylic acid."

From the standpoint of easily homogeneously dissolving the polymer in the anhydrous polyhydric alcohol, the ethylenically unsaturated carboxylic acid monomer is preferably (meth)acrylic acid.

In the present invention, the polymer contains a unit that is a salt formed by the neutralization of the carboxylic acid of the ethylenically unsaturated carboxylic acid unit ("ethylenically unsaturated carboxylic acid salt unit" below) in addition to the ethylenically unsaturated carboxylic acid unit.

The ethylenically unsaturated carboxylic acid salt unit has, for example, a structure in which the ethylenically unsaturated carboxylic acid unit is neutralized with a base. The carboxylic acid salt in the ethylenically unsaturated carboxylic acid salt unit can be any salt. Examples include alkali metal salts of carboxylic acid (e.g., a sodium salt), organic amine salts, and ammonium salts.

The ethylenically unsaturated carboxylic acid salt unit can be formed, for example, by polymerizing a monomer that has formed a salt by neutralization of an ethylenically unsaturated carboxylic acid monomer beforehand ("ethylenically unsaturated carboxylic acid salt monomer" below). Alternatively, an ethylenically unsaturated carboxylic acid salt unit can also be formed by a method of neutralizing the polymer containing an ethylenically unsaturated carboxylic acid unit to convert the ethylenically unsaturated carboxylic acid unit into an ethylenically unsaturated carboxylic acid salt unit.

The polymer may contain a repeating unit other than the ethylenically unsaturated carboxylic acid unit and the ethylenically unsaturated carboxylic acid salt unit as long as the effects of the present invention are not impaired. In the total structural units of the polymer, the ethylenically unsaturated carboxylic acid unit and the ethylenically unsaturated carboxylic acid salt unit are present in an amount of preferably 80 mass% or more, more preferably 90 mass% or more, and particularly preferably 99 mass% or more. The polymer may be formed only of the ethylenically unsaturated carboxylic acid unit and the ethylenically unsaturated carboxylic acid salt unit.

The polymer may have a crosslinked structure or a non-crosslinked structure as long as the effects of the present invention are not impaired. For a polymer that has a crosslinked structure, the crosslinking agent for forming a crosslinked structure can be any crosslinking agent and can be selected from a wide range of known crosslinking agents. Examples of crosslinking agents include water-soluble crosslinking agents.

Water-soluble crosslinking agents include N,N'-methylenebis acrylamide, ethylene glycol dimethacrylate, polyethylene glycol dimethacrylate, and water-soluble sucrose allyl ether. These water-soluble crosslinking agents can be used singly or in a combination of two or more. Of these, water-soluble sucrose allyl ether is preferable for use as a water-soluble crosslinking agent. The degree of etherification of water-soluble sucrose allyl ether can be, for example, 2.0 to 3.5, and is preferably 2.2 to 3.2. As used here, the degree of etherification refers to the average of molar ratios of allyl ether groups to sucrose. The degree of etherification can be determined by reacting hydroxyl groups remaining in sucrose allyl ether with acetic anhydride in pyridine, and calculating the degree of etherification from the amount of acetic anhydride consumed during this reaction. The method for forming water-soluble sucrose allyl ether can also be any method, and can be selected, for example, from a wide range of known production methods.

In the present invention, the polymer has a degree of neutralization of 20 to 75%. Specifically, the polymer is a partially neutralized product.

As used here, the degree of neutralization refers to the content (mol%) of the neutralized repeating unit in the total repeating structural units that constitute the acid component of the polymer. For example, the degree of neutralization of the polymer formed only of the ethylenically unsaturated carboxylic acid unit and the ethylenically unsaturated carboxylic acid salt unit refers to the proportion (mol%) of the ethylenically unsaturated carboxylic acid salt unit of the total number of mols of the ethylenically unsaturated carboxylic acid unit and the ethylenically unsaturated carboxylic acid salt unit.

A polymer having a degree of neutralization of less than 20% is poorly dispersed in the dispersion according to the present invention and may agglomerate over time. This prevents the polymer from homogeneously dissolving in a polyhydric alcohol, resulting in a failure to obtain a desired gelled composition.

A polymer having a degree of neutralization of more than 75% (excluding 75%) is insoluble in the polyhydric alcohol in the gelled composition according to the present invention, resulting in a failure to obtain a desired gelled composition.

The polymer has a degree of neutralization of particularly preferably 20% or more and 40% or less.

The polymer can be a random copolymer, a block copolymer, an alternating copolymer, or a graft copolymer, as long as the polymer contains an ethylenically unsaturated carboxylic acid unit and an ethylenically unsaturated carboxylic acid salt unit.

The polymer cab also have any mass average molecular weight, and the mass average molecular weight can be suitably adjusted as long as the effects of the present invention are not impaired.

In the present invention, the method for producing the polymer can be any method, and can be selected, for example, from a wide range of known polymerization methods. Examples of polymerization methods include reversed-phase suspension polymerization, solution polymerization, and dispersion polymerization.

For example, the polymer can be produced by subjecting a monomer that contains the ethylenically unsaturated carboxylic acid monomer and the ethylenically unsaturated carboxylic acid salt monomer to a known polymerization reaction (e.g., a known radical polymerization). The monomer may optionally contain a crosslinking agent such as the water-soluble crosslinking agent described above. In this case, the obtained polymer can have a crosslinked structure. The content of the crosslinking agent can be any content, and is, for example, 0.1 to 5 parts by mass per 100 parts by mass of the ethylenically unsaturated carboxylic acid monomer.

Alternatively, the polymer can be produced by subjecting a monomer that contains the ethylenically unsaturated carboxylic acid monomer to a known polymerization reaction (e.g., a known radical polymerization) and then performing neutralization treatment. In this case, the method for performing neutralization treatment can be any method and can be selected from a wide range of known methods. In the neutralization treatment, an alkali can be used, and the alkali can be of any type. Examples of alkalis include ammonia, organic amines, and alkali metal hydroxides.

Examples of organic amines include methylamine, ethylamine, diethylamine, triethylamine, diethanolamine, and triethanolamine. Examples of alkali metal hydroxides include sodium hydroxide, potassium hydroxide, and lithium hydroxide. Ammonia can be used in the form of ammonia gas or aqueous ammonia solution. These bases may be used singly or in combination of two or more.

In the present invention, the polymer may be obtained from commercially available products.

In the gelled composition according to the present invention, the polymer is present in a dissolved state in a polyhydric alcohol.

In the dispersion according to the present invention, the polymer is present in a dispersed state in a polyhydric alcohol.

In the dispersion according to the present invention, the polymer is in the form of spherical particles. As used here, "spherical particles" refers to perfect spheres or elliptical spheres, and not those with an irregular shape. The perfect sphere has an aspect ratio (maximum diameter/minimum diameter) of 1. A polymer in the form of elliptical spheres may have any aspect ratio, and the aspect ratio may be, for example, more than 1 and 1.5 or less.

The mean particle size of the polymer for the dispersion according to the present invention can be any size. For example, from the standpoint of easily maintaining the excellent dispersing state of the polymer in the dispersion for a prolonged time, the mean particle size of the polymer can be 5 to 100 µm. The mean particle size refers to the value as determined by dispersing the polymer in n-hexane and measuring the dispersed polymer with a laser diffraction particle size distribution analyzer. The analyzer for use may be an SALD2000 (manufactured by Shimadzu Corporation).

### Anhydrous Polyhydric Alcohol

In the present invention, the anhydrous polyhydric alcohol is a water-free polyhydric alcohol. However, the anhydrous polyhydric alcohol may contain water that is inevitably present. Specifically, the anhydrous polyhydric alcohol in the present invention means a polyhydric alcohol to which water is not intentionally added. For example, inevitable water is present in an amount of 0.5 mass% or less, and preferably 0.1 mass% or less in the anhydrous polyhydric alcohol.

The anhydrous polyhydric alcohol can be of any type, and examples include a wide range of known anhydrous polyhydric alcohols. For example, the anhydrous polyhydric alcohol can be at least one member selected from the group consisting of glycerol, 1,3-butanediol (butylene glycol), propylene glycol, dipropylene glycol, diglycerol, isoprene glycol, and 1,2-pentanediol. From the standpoint of easily homogeneously dissolving the polymer, the anhydrous polyhydric alcohol preferably contains glycerol. If the anhydrous polyhydric alcohol contains glycerol, the content of glycerol in the anhydrous polyhydric alcohol is preferably 80 mass% or more, more preferably 90 mass% or more, still more preferably 95 mass% or more, and particularly preferably 99 mass% or more.

The anhydrous polyhydric alcohol can be produced by a known method or may be obtained from commercially available products.

### Gelled Composition

The gelled composition according to the present invention may contain additives in addition to the polymer and the polyhydric alcohol as long as the effects of the present invention are not impaired. Additives include pH adjusters, light stabilizers, antioxidants, preservatives, colorants, and antifungal agents. The gelled composition may contain one additive or two or more additives. If the gelled composition contains one or more additives, the content thereof is 5 mass% or less, and preferably 1 mass% or less based on the total mass of the gelled composition.

In the gelled composition according to the present invention, the polymer can be of any content. From the standpoint of easily homogeneously dissolving the polymer in the anhydrous polyhydric alcohol, the polymer in the gelled composition is preferably present in an amount of 0.05 to 0.95 mass%, and particularly preferably 0.1 to 0.5 mass% based on the total mass of the anhydrous polyhydric alcohol and the polymer.

In the gelled composition according to the present invention, the polymer is homogeneously dissolved in the anhydrous polyhydric alcohol, and the polymer can play a role of thickening agent for the anhydrous polyhydric alcohol.

Because the polymer is homogeneously dissolved in the anhydrous polyhydric alcohol, the gelled composition according to the present invention can have a variety of applications. In particular, due to the anhydrous polyhydric alcohol present as a base, the gelled composition according to the present invention can be suitably used in cosmetic materials, such as skin-care cosmetics, body-care cosmetics, fragrance cosmetics, massage gels, sheet masks, scrub cosmetic materials, and nourishing cosmetic materials.

### Dispersion

In the dispersion according to the present invention, the polymer is present in a dispersed state in the anhydrous polyhydric alcohol, and can maintain a stable dispersed state.

In the dispersion according to the present invention, the polymer can be of any content. From the standpoint of easily dispersing the polymer in the anhydrous polyhydric alcohol while not dissolving the polymer in the anhydrous polyhydric alcohol and making the dispersibility excellent, the polymer in the dispersion is present in an amount of preferably 1.0 to 16.0 mass%, and particularly preferably 2.0 to 9.1 mass% based on the total mass of the anhydrous polyhydric alcohol and the polymer.

Because the polymer in the dispersion according to the present invention is stably dispersed in the anhydrous polyhydric alcohol, further adding the anhydrous polyhydric alcohol to the dispersion makes it easier for the polymer to homogeneously dissolve in the anhydrous polyhydric alcohol. Accordingly, the gelled composition according to the present invention is easily prepared. If an attempt is made to dissolve the polymer without dispersing the polymer in the anhydrous polyhydric alcohol, the polymer cannot homogeneously dissolve in the anhydrous polyhydric alcohol, leaving swelled polymer and aggregates. This makes it difficult to obtain a gelled composition in which the polymer is homogeneously dissolved. However, the use of the dispersion according to the present invention makes it unlikely for the polymer to inhomogeneously dissolve, and thus makes it easier to obtain a desired gelled composition. Specifically, a desired gelled composition is more easily obtained by using the method of first dispersing the polymer in an anhydrous polyhydric alcohol, and then further adding the anhydrous polyhydric alcohol to dissolve the polymer.

Thus, the dispersion according to the present invention is suitable as a starting material for producing a gelled composition that contains the polymer dissolved in the anhydrous polyhydric alcohol.

### Method for Producing Gelled Composition

The method for producing the gelled composition according to the present invention can be any method and can be selected from a wide range of known production methods.

In an embodiment, the method for producing the gelled composition according to the present invention may include the following first step and second step.
First step: preparing a dispersion of a polymer containing an ethylenically unsaturated carboxylic acid unit in an anhydrous polyhydric alcohol, and
Second step: mixing the dispersion with an anhydrous polyhydric alcohol to prepare a solution of the polymer in the polyhydric alcohol.

The dispersion for use in the first step is the dispersion according to the present invention described above. Thus, the polymer for use in preparing the dispersion of the first step is the same as the polymer contained in the dispersion according to the present invention. Specifically, the polymer for use in the first step is a partially neutralized product that has a degree of neutralization of 20 to 75%, and is in the form of spherical particles. The anhydrous polyhydric alcohol for use in preparing the dispersion of the first step is the anhydrous polyhydric alcohol contained in the dispersion according to the present invention.

The method for preparing the dispersion in the first step can be any method. For example, the dispersion can be obtained by mixing a polymer with an anhydrous polyhydric alcohol and stirring the mixture.

In the first step, the proportion of the polymer for use is preferably 1.0 to 16.0 mass% based on the total mass of the anhydrous polyhydric alcohol and the polymer. In this case, the polymer can be more stably dispersed in the obtained dispersion. In the first step, the proportion of the polymer for use is particularly preferably 2.0 to 9.1 mass% based on the total mass of the anhydrous polyhydric alcohol and the polymer.

In the first step, the method for mixing the polymer with the anhydrous polyhydric alcohol and the method for stirring the mixture can be any method, and each can be selected from a wide range of known methods.

In the second step, the dispersion obtained in the first step and an anhydrous polyhydric alcohol are mixed to prepare a solution of the polymer in the polyhydric alcohol. The anhydrous polyhydric alcohol for use in the second step may be of the same type as or different type from the anhydrous polyhydric alcohol contained in the dispersion. From the standpoint of easily dissolving the polymer in the anhydrous polyhydric alcohol, the anhydrous polyhydric alcohol for use in the second step is preferably the same as the anhydrous polyhydric alcohol contained in the dispersion.

The proportion of the anhydrous polyhydric alcohol further added in the second step can be any proportion as long as the polymer in the dispersion is dissolved. For example, the amount of the further added anhydrous polyhydric alcohol can be adjusted so that the content of the polymer in the gelled composition ultimately obtained in the second step is 0.05 to 0.95 mass% (preferably, 0.1 to 0.5 mass%) based on the total mass of the anhydrous polyhydric alcohol and the polymer (the mass of the anhydrous polyhydric alcohol is the total mass of the anhydrous polyhydric alcohol contained in the dispersion and the further added anhydrous polyhydric alcohol in the second step).

The method for mixing the dispersion with the anhydrous polyhydric alcohol in the second step can be any method, and can be selected, for example, from a wide range of known mixing methods.

The mixture obtained by mixing the dispersion with the anhydrous polyhydric alcohol may be subjected to heat treatment. The conditions for heat treatment can be any conditions. For example, the mixture can be heated at 60 to 90°C. The heat treatment may be performed in multiple stages; specifically, the method of heating the mixture at 60 to 70°C for a predetermined time period, and then increasing the heating temperature to heat the mixture at 80 to 90°C for a predetermined time period can also be used. The heating time can be any time and can be adjusted to a suitable range as long as the polymer can be dissolved in the anhydrous polyhydric alcohol.

Due to the first step and the second step, the method for producing the gelled composition according to the present invention can easily produce a gelled composition. In particular, due to the use of the dispersion according to the present invention in the method for producing the gelled composition according to the present invention, the polymer can be easily homogeneously dissolved in the resulting gelled composition. Due to the first step performed, the ultimately obtained gelled composition is unlikely to contain a remaining swelled polymer and aggregates.

Additionally, due to the first step performed, the method for producing a gelled composition according to the present invention can cause the polymer to be quickly dissolved in the anhydrous polyhydric alcohol. Thus, this method can shorten the total production time compared with a method for producing a gelled composition without performing the first step. Additionally, because the dispersion exhibits excellent dispersibility of the polymer, the production method according to the present invention can reduce, for example, phenomena such as the adhesion of the polymer on the container wall and is thus excellent in handling, compared with a method for producing a gelled composition without preparing a dispersion.

### Examples

The following describes the present invention in more detail with reference to Examples. However, the present invention is not limited to the embodiments of these Examples.

### Synthesis of Water-Soluble Crosslinking Agent

A stirrer, a reflux condenser, and a dropping funnel were attached to a 1000-mL separable flask. In this flask, 48 g of sodium hydroxide was dissolved in 144 g of water. Subsequently, 136.8 g of sucrose was added thereto, and the mixture was stirred at 70 to 85°C for 120 minutes, thereby obtaining an aqueous solution of alkaline sucrose. 145.2 g of allyl bromide was added dropwise to the obtained aqueous solution of alkaline sucrose at 70 to 85°C over the course of 1.5 hours, and then the mixture was reacted at 80°C for 3 hours to allyl-etherify the sucrose. After cooling, 440 g of water was added thereto, and excessive oil was removed with a separatory funnel, thereby obtaining an aqueous solution of crude sucrose allyl ether. Hydrochloric acid was added to the obtained crude sucrose allyl ether to adjust the pH to 6 to 8, and water was removed with a rotary evaporator until the mass of the aqueous solution reached 480 g. Subsequently, 200 g of ethanol was added thereto, and salts, such as sodium bromide, (by-products) were precipitated, followed by removing the precipitates from the aqueous solution through filtration. Further, excessive water was removed from the aqueous solution with an evaporator, thereby obtaining 166 g of sucrose allyl ether with a degree of etherification of 2.4.

### Synthesis of Polymer Containing Ethylenically Unsaturated Carboxylic Acid Unit

### Production Example 1

A stirrer, a reflux condenser, and a dropping funnel were attached to a 500-mL separable flask. 72 g of acrylic acid and water were added to the flask to prepare 90 g of an 80 mass% aqueous acrylic acid solution. While this aqueous acrylic acid solution was cooled, 27 g of a 30 mass% aqueous sodium hydroxide solution was added dropwise thereto to prepare an aqueous acrylic-acid-neutralized solution with a degree of neutralization of 20%. Subsequently, 0.32 g of the sucrose allyl ether obtained above and 0.04 g of 2,2'-azobis(2-methylpropionamidine) dihydrochloride (Wako Pure Chemical Industries, Ltd., V-50) were added thereto, thereby preparing an aqueous solution of an ethylenically unsaturated carboxylic acid monomer.

Separately, 330 g of n-heptane was added to a 2000-mL separable flask equipped with a stirrer, a reflux condenser, a dropping funnel, and a nitrogen gas inlet tube, and 2.7 g of sorbitan monostearate (NOF Corporation, NONION SP-60R) was further added thereto, followed by dispersing and dissolving it in n-heptane. Subsequently, the aqueous solution of an ethylenically unsaturated carboxylic acid monomer prepared in advance was added thereto. To remove the oxygen present in the atmosphere in the reactor, in the starting materials, and in the solvent, nitrogen gas was blown into the solution. While the inside of the system was replaced with nitrogen, a reaction was performed for 1 hour with the bath temperature maintained at 60°C, with stirring at a rotational frequency of 1000 rotations/minute. After completion of the reaction, water and n-heptane were distilled off, thereby obtaining a polymer of acrylic acid and a sodium salt thereof. The obtained polymer had a perfect spherical shape.

### Production Example 2

A polymer was prepared in the same manner as in Production Example 1 except that the amount of the 30 mass% aqueous sodium hydroxide solution was changed to 54 g (degree of neutralization: 40%). The obtained polymer was in the form of perfect spheres.

### Production Example 3

A polymer was prepared in the same manner as in Production Example 1 except that the amount of the 30 mass% aqueous sodium hydroxide solution was changed to 100 g (degree of neutralization: 75%). The obtained polymer was in the form of perfect spheres.

### Production Example 4

A polymer was prepared in the same manner as in Production Example 1 except that the aqueous acrylic acid solution was not neutralized (degree of neutralization: 0%). The obtained polymer was in the form of perfect spheres.

### Production Example 5

Polyacrylic acid particles that have a degree of neutralization of 0% and an irregular shape (AQUPEC HV-505E, produced by Sumitomo Seika Chemicals Co., Ltd) were prepared.

### Production Example 6

Polyacrylic acid particles that have a degree of neutralization of 70% and an irregular shape (PNC400, produced by 3V Sigma) were prepared.

### Production Example 7

Polyacrylic acid Na particles that have a degree of neutralization of 100% and an irregular shape (sodium polyacrylate, produced by Wako Pure Chemical Industries, Ltd.) were prepared.

### Preparation and Evaluation of Gelled Composition

### Example 1

In a vessel, 0.3 parts by mass of the polymer obtained in Production Example 1 and 5 parts by mass of anhydrous glycerol were mixed to prepare a dispersion of the polymer in the anhydrous glycerol (first step). This dispersion obtained in the first step was visually observed for the dispersibility immediately after the preparation and the dispersibility after 10 minutes from the preparation. In both cases, the dispersing state of the polymer was excellent, and no lumps were confirmed.

To the dispersion obtained in the first step, 94.7 parts by mass of anhydrous glycerol was further added to prepare a mixture, and the mixture was continuously stirred at 90°C for 1 hour. This caused the polymer to dissolve in glycerol, thereby obtaining a gelled composition (second step). Visual observation confirmed that this gelled composition obtained in the second step contained no swelled polymer and aggregates, that the polymer was homogeneously dissolved, and that the composition was colorless and transparent.

### Example 2

A gelled composition was prepared in the same manner as in Example 1 except that the polymer obtained in Production Example 1 was changed to the polymer obtained in Production Example 2. The dispersion obtained in the first step was visually observed for the dispersibility immediately after the preparation and the dispersibility after 10 minutes from the preparation. In both cases, the dispersing state of the polymer was excellent, and no lumps were confirmed.

Visual observation confirmed that the gelled composition obtained in the second step contained no swelled polymer and aggregates, that the polymer was homogeneously dissolved, and that the composition was colorless and transparent.

### Example 3

A gelled composition was prepared in the same manner as in Example 1 except that the polymer obtained in Production Example 1 was changed to the polymer obtained in Production Example 3. The dispersion obtained in the first step was visually observed for the dispersibility immediately after the preparation and the dispersibility after 10 minutes from the preparation. In both cases, the dispersing state of the polymer was excellent, and no lumps were confirmed.

Visual observation confirmed that the gelled composition obtained in the second step contained no swelled polymer and aggregates, that the polymer was homogeneously dissolved, and that the composition was colorless and transparent.

### Comparative Example 1

A gelled composition was prepared in the same manner as in Example 1 except that the polymer obtained in Production Example 1 was changed to the polymer obtained in Production Example 4. The dispersion obtained in the first step was visually observed for the dispersibility immediately after the preparation and the dispersibility after 10 minutes from the preparation. Although the dispersing state of the polymer immediately after preparation was excellent, the dispersion after 10 minutes from preparation contained lumps, showing a poorly dispersing state.

Visual observation also confirmed that a swelled polymer and aggregates were clearly present in the gelled composition obtained in the second step, and that the polymer was inhomogeneously dissolved.

### Comparative Example 2

A gelled composition was prepared in the same manner as in Example 1 except that the polymer obtained in Production Example 1 was changed to the polymer obtained in Production Example 5. The dispersion obtained in the first step was visually observed for the dispersibility immediately after the preparation and the dispersibility after 10 minutes from the preparation. In both cases, lumps were confirmed, and the dispersing state was poor.

Visual observation also confirmed that a swelled polymer and aggregates were clearly present in the gelled composition obtained in the second step, and that the polymer was inhomogeneously dissolved.

### Comparative Example 3

A gelled composition was prepared in the same manner as in Example 1 except that the polymer obtained in Production Example 1 was changed to the polymer obtained in Production Example 6. The dispersion obtained in the first step was visually observed for the dispersibility immediately after the preparation and the dispersibility after 10 minutes from the preparation. In both cases, lumps were confirmed, and the dispersing state was poor.

Visual observation also confirmed that a swelled polymer and aggregates were clearly present in the gelled composition obtained in the second step, and that the polymer was inhomogeneously dissolved.

### Comparative Example 4

A gelled composition was prepared in the same manner as in Example 1 except that the polymer obtained in Production Example 1 was changed to the polymer obtained in Production Example 7. The dispersion obtained in the first step was visually observed for the dispersibility immediately after the preparation and the dispersibility after 10 minutes from the preparation. In both cases, the polymer was well dispersed, and no lumps were confirmed.

However, visual observation confirmed that the polymer remained without being swelled and dissolved in the gelled composition obtained in the second step.

## Claims

1. A gelled composition comprising
a polymer containing an ethylenically unsaturated carboxylic acid unit, and
an anhydrous polyhydric alcohol, wherein the polymer is a partially neutralized product that has a degree of neutralization of 20 to 75%, and the polymer is dissolved in the anhydrous polyhydric alcohol.

2. The gelled composition according to claim 1, wherein the polymer is present in an amount of 0.05 to 0.95 mass% based on the total mass of the anhydrous polyhydric alcohol and the polymer.

3. A dispersion comprising
a polymer containing an ethylenically unsaturated carboxylic acid unit, and
an anhydrous polyhydric alcohol, wherein the polymer is a partially neutralized product that has a degree of neutralization of 20 to 75%, the polymer is in the form of spherical particles, and the polymer is dispersed in the anhydrous polyhydric alcohol.

4. The dispersion according to claim 3, wherein the polymer is present in an amount of 1.0 to 16.0 mass% based on the total mass of the anhydrous polyhydric alcohol and the polymer.

5. A method for producing the gelled composition of claim 1 or 2, the method comprising
a first step of preparing a dispersion of a polymer containing an ethylenically unsaturated carboxylic acid unit in an anhydrous polyhydric alcohol, and
a second step of mixing the dispersion with an anhydrous polyhydric alcohol to prepare a solution of the polymer in the polyhydric alcohol,
wherein the polymer for use in the first step is a partially neutralized product that has a degree of neutralization of 20 to 75%, and the polymer for use in the first step is in the form of spherical particles.

6. The method according to claim 5, wherein in the first step, the proportion of the polymer for use is 1.0 to 16.0 mass% based on the total mass of the anhydrous polyhydric alcohol and the polymer.
